# EUROPEAN PATENT SPECIFICATION

(11) **EP 1 322 455 B1**
(45) Date of publication and mention of the grant of the patent: **20.10.2004**
(21) Application number: 01969943.8
(22) Date of filing: 21.09.2001
(51) Int. Cl.: B29C 35/02, G01N 33/44, B29C 70/54

(54) **PREDICTING BEHAVIOUR OF A MOULDED COMPOSITE COMPONENT**
VERHALTENSVORHERSAGE EINES GEFORMTEN VERBUNDBAUTEILS
PREDICTION DU COMPORTEMENT D'UN COMPOSANT COMPOSITE MOULE

(30) Priority: 03.10.2000 GB 0024121
(43) Date of publication of application: 02.07.2003
(73) Proprietor: Airbus UK Limited, Bristol, BS99 7AR (GB)
(72) Inventor: BARRELLON, Hervé, Chippenham SN14 0HF (GB)
(74) Representative: Edis, Ronald Malcolm
(86) International application number: PCT/GB2001/004205
(87) International publication number: WO 2002/028611

(56) References cited:
- US-A- 5 770 155

## Description

The invention relates to a method of predicting material behaviour and is primarily concerned with the prediction of a phenomenon known as "spring back" in a moulded fibre/resin composite component.

Spring back is an angular distortion of a composite component relative to its mould once it has cured. For example, in the case of a moulded L-shaped component, the limbs of the component which are moulded at 90° tend to move (spring back) relative to each other when the cured component is removed from the mould. It has been found that the spring back is primarily due to a difference in chemical and thermal properties of the fibre compared to that of the resin during curing of the composite.

Where a composite component is thin and fairly flexible, for example in a fairing for use on an aircraft, the problem of spring back can be overcome simply by bending the component slightly into the correct shape during installation. However, where a structural component is involved the problem of spring back becomes more acute as the size and thickness of the moulded component will normally make such bending impossible. Therefore, far more accuracy is required in making the moulding such that once it is cured, spring back will take the component to its correct shape. Where such accuracy is involved, the standard approach is a trial and error method. Depending on the accuracy required, the value of angular distortion on leaving the mould will be estimated and the component will be moulded accordingly. If, on leaving the mould, the component is not within manufacturing tolerances, the mould will be adjusted or even rebuilt and another component moulded. It will be appreciated that for very large components, for example, a large structural component for an aircraft, such a trial and error method is costly and inefficient.

An object of the present invention is to provide a method of predicting behaviour of a moulded composite component which will enable spring back to be predicted accurately without the inefficiency of the trial and error method currently used.

According to one aspect of the invention there is provided a method of predicting behaviour such as spring back of a moulded composite component made from fibre and resin materials, the method comprising determining the temperature at which the resin passes from a liquid state to a solid state, constructing a finite element model, producing from a fibre and resin corresponding to those from which the component is made a plurality of elements smaller than the component, determining for each element at different temperatures the difference between variation in size caused thermally and variation in size caused chemically, taking the information obtained and adjusting the finite element model in response thereto to enable a coefficient of thermal expansion to be determined, and using the finite element model to provide an indication of the behaviour of cured moulded composite components on being removed from the mould.

Particular embodiments of the method are the subject of dependent claims 2 to 4.

Preferably, the elements are miniature versions of at least a part of the component and for the most reliable results should be precise miniature versions. Each element may be identical to the other.

The method preferably involves producing from ten to fifteen of the said elements to enable data derived therefrom to be averaged to provide optimum accuracy. Preferably, said elements are produced in groups of three identical elements to provide statistical repeatability. For each group, the geometry of the corner (e.g. thickness, corner radius, lay up) may be varied.

According to a second aspect of the invention there is provided apparatus for performing the method of the first aspect of the invention or any of the subsidiary clauses relating thereto comprising cure monitoring means for determining the temperature at which the resin passes from a liquid state to a solid state, a plurality of elements smaller than the component to be moulded, the elements being made from fibre and resin materials corresponding to those from which the component is made, means for determining for each element at different temperatures the difference between variation in size caused thermally and variation in size caused chemically to enable the coefficient of thermal expansion to be determined, and a finite element model to which the determined parameters can be applied to provide an indication of the behaviour of the cured moulded composite on being removed from the mould.

Particular embodiments of the apparatus are the subject of dependent claims 6 to 8.

The cure monitoring means is preferably a dielectric cure monitoring system. Such a system is of a kind which can be used to monitor chemical reaction in the resin during curing.

The finite element model is preferably a three dimensional model which preferably models discreet plies of fibre used to form the component, that is a three dimensional ply by ply finite element model.

A method and apparatus in accordance with the invention will now be described by way of example with reference the accompanying drawings in which:-
Figure 1 shows a cross section of moulded composite component in a mould,
Figure 2 shows the component removed from the mould and illustrates the effect of spring back,
Figure 3 shows steps in a method in accordance with the invention for predicting spring back in a moulded component,
Figure 4 shows a mould for use in forming the component using the method of Figure 3 and
Figure 5 shows a cross section of the component made in the mould of Figure 4.
Figure 6 shows the size of an element (E) to be produced from the component of Figures 2 and 5.

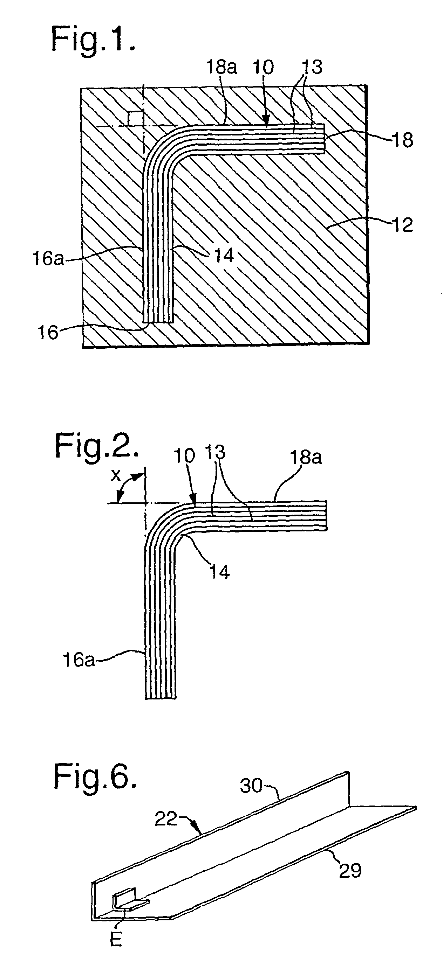

Looking at Fig 1, an L-shaped component 10 is formed in a mould 12 as a composite using a lay up of carbon fibre plies 13 impregnated with resin 14. The mould 12 is made such that respective limbs 16, 18 of the component 10 have external surfaces 16a, 18a at right angles to each other.

The resin 14 is allowed to cure in the mould 12 and the component 10 is then removed as shown in Fig 2. The curing of the resin 14 coupled with other properties, in particular the orientation of the plies 13, contribute to spring back of the limbs 16, 18 which, as shown in Fig 2 results in the surfaces 16a, 18a no longer being at right angles but at a larger angle x relative to each other.

The present invention is aimed at predicting spring back in a moulded composite component.

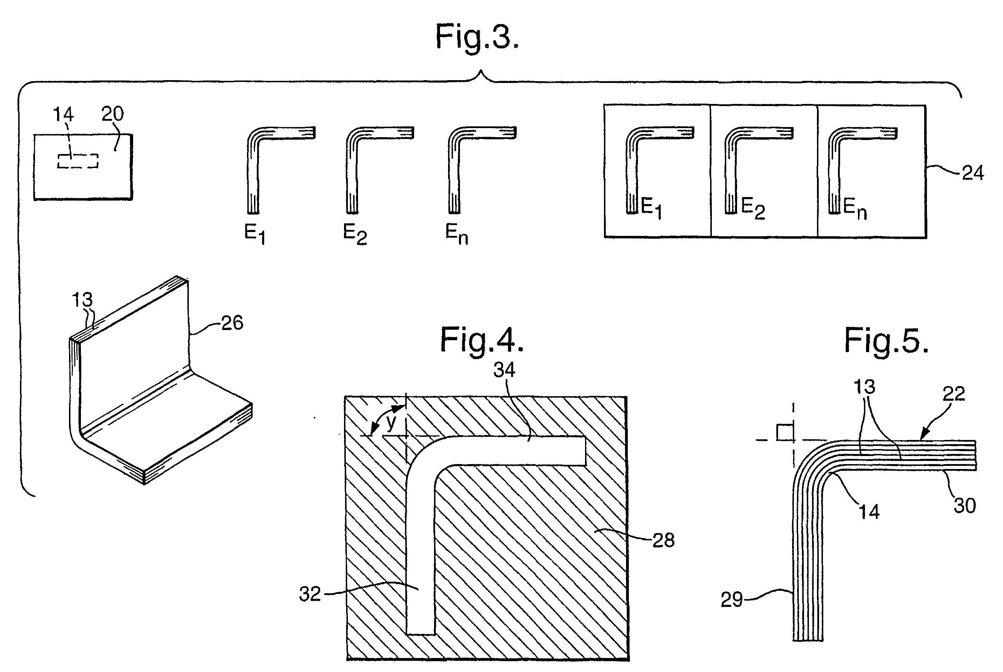

Looking at Fig 3, the method involves taking a sample of the type of resin 14 used in the composite, for example Hexcel 8552 - AS4, and using a dielectric cure monitoring system 20 to determine what is known as the "stress free temperature", that is the temperature at which the resin 14 passes from a liquid state to a solid state or gel point. Such a cure monitoring system 20 is of a known kind which can be used to monitor chemical reaction in the resin during curing and to determine the chemical shrinkage.

The data obtained is then used to construct a three dimensional ply by ply finite element model.

A number of composite elements E1, E2......En are produced. In a preferred embodiment, between ten and fifteen of such elements will be produced. Each composite element E1 to En is an accurately formed miniature version of the component (indicated at 22 in Figure 6) in which spring back is to be predicted. The component 22, like the component 10 in Fig 1 is made up of carbon fibre plies 13 impregnated with resin 14. Each of the elements E1 to En is measured accurately at different temperatures at a measurement station 24 in order to determine thermal shrinkage separately for each component. The measurements taken from the elements E1 to En manufactured initially are used to calibrate the three dimensional ply by ply finite element model 26, i.e. the model is made to match the data obtained, thereby enabling the "true" coefficient of thermal expansion to be determined for the elements. The advantage of using ten to fifteen elements is that the data required can be deduced very accurately.

The finite element model 26 is a computer generated three dimensional model which has been set up to model the shape of the component 22 in which spring back is to be predicted and which models each discreet ply 13 of the component 22 and the particular orientation thereof. The measurements based on the elements E1 to En enable the finite model to be controlled whereby a complementary shaped mould 28 (see Fig 4) can be constructed to produce the final component 22 in such a way that on removal of the component from the mould 28, the spring back in the component 22 will position limbs 29, 30 thereof precisely as required in the cured component. For example, in the mould 28 shown, sections 32, 34 thereof which mould the respective limbs 29, 30 are set at an angle y less than 90° so that the when the moulded component 22 springs back on removal from the mould, the limbs 28, 30 will lie at right angles to each other. When moulding a subsequent and different L-shaped component, data derived from measuring a miniature set of elements based on the subsequent component and the ply orientation thereof can be used as inputs to the calibrated finite element model 26 to predict the spring back and enable the mould to be shaped accordingly so as to compensate for spring back in the final cured component.

## Claims

1. A method of predicting behaviour such as spring back of a moulded composite component (22) made from fibre and resin materials (13, 14), the method comprising determining the temperature at which the resin (14)passes from a liquid state to a solid state, constructing a finite element model (26), producing from a fibre and resin corresponding to those from which the component is made a plurality of elements (E₁ to Eₙ) smaller than the component, determining for each element (E₁ to Eₙ) at different temperatures the difference between variation in size caused thermally and variation in size caused chemically, taking the information obtained and adjusting the finite element model (26) in response thereto to enable a coefficient of thermal expansion to be determined, and using the finite element model (26) to provide an indication of the behaviour of the cured moulded composite component (22) on being removed from the mould (28).

2. A method according to claim 1 comprising forming the elements (E₁ to Eₙ) as miniature versions of the component.

3. A method according to claim 1 or 2 comprising forming the elements (E₁ to Eₙ) so as to be identical to each other.

4. A method according to 1, 2 or 3 comprising producing from ten to fifteen of the said elements (E₁ to Eₙ) to enable data derived therefrom to provide optimum accuracy.

5. Apparatus for performing a method of predicting behaviour such as spring back of a moulded composite component (22) made from fibre and resin materials (13, 14), the apparatus comprising cure monitoring means for determining the temperature at which the resin passes from a liquid state to a solid state, a plurality of elements (E₁ to Eₙ) smaller than the component to be moulded, the elements being made from fibre and resin materials corresponding to those from which the component is made, means for determining for each element at different temperatures the difference between variation in size caused thermally and variation in size caused chemically to enable the coefficient of thermal expansion to be determined, and a finite element model (26) to which the determined parameters can be applied to provide an indication of the behaviour of the cured moulded composite (22) on being removed from the mould (28).

6. Apparatus according to claim 5 in which the cure monitoring means is a dielectric cure monitoring system.

7. Apparatus according to claim 5 or 6 in which the finite element model is a three dimensional model.

8. Apparatus according to claim 5, 6 or 7 in which the finite element model models discreet plies from which the component is made.

## Patentansprüche

1. Verfahren zur Vorhersage des Verhaltens, beispielsweise des Rückfedems, eines gegossenen Verbundbauteils (22), welcher aus Fasern und Kunststoffmaterialien (13, 14) besteht, wobei das Verfahren die folgenden Schritte umfasst: es wird die Temperatur bestimmt, bei der der Kunststoff (14) aus dem flüssigen Zustand in einen festen Zustand übergeht; es wird ein finites Elementenmodell (26) konstruiert; es werden aus Faser- und Kunststoffmaterialien entsprechend denen, aus denen der Bauteil hergestellt werden soll, mehrere Elemente (E₁ bis Eₙ) erzeugt, die kleiner sind als der Bauteil; es wird für jedes Element (E₁ bis Eₙ) bei verschiedenen Temperaturen die Differenz bestimmt, die zwischen der thermisch verursachten Größenveränderung und der chemisch verursachten Größenveränderung besteht; es wird die erhaltene Information abgenommen und das finite Elementenmodell (26) demgemäß eingestellt, um einen thermischen Ausdehnungskoeffizienten zu bestimmen, und es wird das finite Elementenmodell (26) benutzt, um eine Anzeige des Verhaltens des ausgehärteten gegossenen Verbundbauteils (22) nach Entnahme aus der Form (28) zu erhalten.

2. Verfahren nach Anspruch 1, bei welchem die Elemente (E₁ bis Eₙ) als Miniaturversionen des Bauteils geformt werden.

3. Verfahren nach den Ansprüchen 1 oder 2, bei welchem die Elemente (E₁ bis Eₙ) so geformt werden, dass sie einander identisch sind.

4. Verfahren nach den Ansprüchen 1, 2 oder 3, bei welchem zehn bis fünfzehn der Elemente (E₁ bis Eₙ) erzeugt werden, um durch die hiervon abgenommenen Daten eine optimale Genauigkeit zu erzeugen.

5. Vorrichtung zur Durchführung des Verfahrens zur Vorhersage des Verhaltens, beispielsweise des Rückfederns, eines gegossenen Verbundbauteils (22), welcher aus Fasern und Kunststoffmaterial (13, 14) besteht, wobei die Vorrichtung die folgenden Teile aufweist: Mittel zur Überwachung der Aushärtung zur Bestimmung der Temperatur, bei der der Kunststoff aus dem flüssigen Zustand in einen festen Zustand übergeht; mehrere Elemente (E₁ bis Eₙ), die kleiner sind als der zu vergießende Bauteil, wobei die Elemente aus den gleichen Fasermaterialien und Kunststoffmaterialien hergestellt sind wie das zu vergießende Bauteil; Mittel, um für jedes Element bei unterschiedlichen Temperaturen die Differenz zwischen der thermisch verursachten Größenveränderung und der chemisch verursachten Größenveränderung zu bestimmen, damit hieraus der thermische Ausdehnungskoeffizient bestimmt werden kann; und ein finites Elementenmodell (26), auf das die bestimmten Parameter angewandt werden können, um eine Anzeige des Verhaltens des ausgehärteten gegossenen Verbundbauteils (22) nach Entnahme aus der Form (28) zu liefern.

6. Vorrichtung nach Anspruch 5, bei welcher die Aushärtungs-Überwachungsmittel aus einem dielektrischen Aushärtungs-Überwachungssystem bestehen.

7. Vorrichtung nach den Ansprüchen 5 oder 6, bei welcher das finite Elementenmodell ein dreidimensionales Modell ist.

8. Vorrichtung nach den Ansprüchen 5, 6 oder 7, bei welcher das finite Elementenmodell diskrete Lagen modelliert, aus denen der Bauteil hergestellt wird.

## Revendications

1. Une méthode de prédiction de comportement tel que le retour élastique d'un composant composite moulé (22) réalisé à partir de matières fibreuse et résineuse (13, 14), la méthode comportant la détermination de la température à laquelle la résine (14) passe d'un état liquide à un état solide, la construction d'un modèle d'élément fini (26), la production à partir d'une fibre et d'une résine correspondant à celles à partir desquelles le composant est réalisé d'une pluralité d'éléments (E₁ à Eₙ) plus petits que le composant, la détermination pour chaque élément (E₁ à Eₙ) à différentes températures de la différence entre la variation de taille d'origine thermique et la variation de taille d'origine chimique, la considération des informations obtenues et l'ajustement du modèle d'élément fini (26) en réponse à celles-ci pour permettre de déterminer un coefficient de dilatation thermique, et l'utilisation du modèle d'élément fini (26) pour fournir une indication du comportement du composant composite moulé (22) durci lorsqu'il est retiré du moule (28).

2. Une méthode selon la revendication 1 comportant la formation des éléments (E₁ à Eₙ) comme des versions miniatures du composant.

3. Une méthode selon la revendication 1 ou la revendication 2 comportant la formation des éléments (E₁ à Eₙ) de façon à ce qu'ils soient identiques les uns aux autres.

4. Une méthode selon les revendications 1, 2 ou 3 comportant la production de dix à quinze desdits éléments (E₁ à Eₙ) afin de permettre aux données dérivées de ceux-ci de fournir une exactitude optimale.

5. Appareil destiné à mettre à exécution une méthode de prédiction de comportement tel que le retour élastique d'un composant composite moulé (22) réalisé à partir de matières fibreuse et résineuse (13, 14), l'appareil comportant un moyen de surveillance de durcissement destiné à déterminer la température à laquelle la résine passe d'un état liquide à un état solide, une pluralité d'éléments (E₁ à Eₙ) plus petits que le composant devant être moulé, les éléments étant réalisés à partir de matières fibreuse et résineuse correspondant à celles à partir desquelles le composant est réalisé, un moyen destiné à déterminer pour chaque élément à différentes températures la différence entre la variation de taille d'origine thermique et la variation de taille d'origine chimique pour permettre de déterminer le coefficient de dilatation thermique, et un modèle d'élément fini (26) auquel les paramètres déterminés peuvent être appliqués pour fournir une indication du comportement du composite moulé (22) durci lorsqu'il est retiré du moule (28).

6. Appareil selon la revendication 5 dans lequel le moyen de surveillance de durcissement est un système de surveillance de durcissement diélectrique.

7. Appareil selon la revendication 5 ou la revendication 6 dans lequel le modèle d'élément fini est un modèle tridimensionnel.

8. Appareil selon les revendications 5, 6 ou 7 dans lequel le modèle d'élément fini modèle des strates discrètes à partir desquelles le composant est réalisé.
